# EUROPEAN PATENT APPLICATION

(11) **EP 4 843 587 A1**
(43) Date of publication of application: **25.11.2026**
(21) Application number: 26170090.0
(22) Date of filing: 31.03.2026
(51) Int. Cl.: A61L 27/20

(54) **METHOD FOR PREPARING VISCOSUPPLEMENT**

(30) Priority: 23.05.2025 CN 202510678053
(71) Applicant: Shanghai Qisheng Biological Preparation Co., Ltd., Shanghai 201106 (CN)
(72) Inventor: WEI, Changzheng, Shanghai, 201106 (CN); ZHU, Jiaxun, Shanghai, 201106 (CN); LU, Tingting, Shanghai, 201106 (CN); WANG, Xiaotong, Shanghai, 201106 (CN); XI, Hongwei, Shanghai, 201106 (CN); JIANG, Lixia, Shanghai, 201106 (CN)
(74) Representative: Bayramoglu et al.

(57) **Abstract**

The present application discloses a method for preparing a viscosupplement, including: a) dissolving a crosslinking agent to produce a crosslinking agent reaction solution, where the crosslinking agent reaction solution includes a buffer system with a pH of 5 to 9, and the crosslinking agent includes two or more amino groups; b) adding carboxymethyl chitin to the crosslinking agent reaction solution, and mixing, where a molar ratio of carboxyl groups in the carboxymethyl chitin to amino groups in the crosslinking agent is 3:2 to 1:2; c) adding a condensing agent to initiate an amidation reaction between the carboxymethyl chitin and the crosslinking agent, where a molar ratio of the carboxyl groups in the carboxymethyl chitin to the condensing agent is 4:1 to 1:2, and the condensing agent is selected from the group consisting of EDC, DMTMM, EDCI, CDMT, or a combination thereof; d) diluting a resulting gel with a buffer to 0.5% to 5% (w/w), where the buffer is selected from the group consisting of a phosphate, a borate, a citrate, or any combination thereof; and e) conducting dialysis with the buffer to produce the viscosupplement, where the method does not include use of ethanol, acetone, or methanol.

## Description

### TECHNICAL FIELD

The present disclosure belongs to the field of general preparation methods for polymer compositions and materials, and specifically relates to a method for preparing a viscosupplement.

### BACKGROUND

Osteoarthritis (OA) is a degenerative joint disease. OA causes pain, swelling, and stiffness, impairing the ability of a person to move freely. OA affects the entire joint, including the periarticular tissues. OA occurs most commonly in the knee joints, hip joints, spine, and hand joints. OA is pathologically characterized by cartilage degeneration, osteophyte formation, subchondral bone remodeling, and synovial inflammation. One of the main pathological features of OA is chronic, low-grade inflammation, primarily caused by joint damage and debris. Such inflammation triggers an innate immune response in the early stage of OA, and ultimately leads to immune cell infiltration and synovial hyperplasia.

Pathophysiological factors influencing OA include mechanical stress on the joint and many individual-specific factors such as genetic susceptibility, ethnicity, nutrition, and gender. Among musculoskeletal disorders, OA is a leading cause of years lived with disability. As OA predominantly affects the elderly (approximately 70% of OA patients are aged 55 years and older), the global prevalence of OA is expected to increase with population aging. OA typically affects those aged from the late 40s to the late 50s, but may also occur in young individuals, including athletes and those with joint injury or trauma. Approximately 60% of OA patients are women.

A joint includes cartilage, synovium, and joint capsule. Articular cartilage basically consists of chondrocytes and extracellular matrix. In articular cartilage, chondrocytes account for only 1%, and the extracellular matrix accounts for 99%, with approximately 3% of which being glycosaminoglycans. Synovial fluid is a transparent, viscous lubricating fluid including mucin-like substances, which is secreted by the synovial membranes of joint bursae and tendon sheaths. As a secretion of body organs and tissues, synovial fluid plays the roles of lubricating and moistening organs and eliminating toxins. If synovial fluid decreases or becomes very viscous, metabolic products will be retained in the body, leading to various disorders. For example, as synovial fluid decreases with age, joints lack lubrication and thus develop degenerative arthritis, bone spurs, osteoporosis, and other conditions due to wear and tear. In addition, long-term lack of synovial fluid in articular cartilage can cause osteonecrosis.

Major treatment approaches for OA include weight management, exercise, nonsteroidal and steroidal anti-inflammatory drugs, opioids, intra-articular platelet-rich plasma, placebos, corticosteroid injections, intra-articular viscosupplements, and surgery.

The progression of degenerative arthritis can be slowed by injecting viscosupplements. Common viscosupplements include hyaluronic acid (also known as sodium hyaluronate) and carboxymethyl chitosan (also known as carboxymethyl glycosaminoglycan and chitin). As a traditional viscosupplement, hyaluronic acid has been used for many years with prominent outcomes.

Carboxymethyl chitin, as a viscosupplement, has a long action duration in the body due to the absence of corresponding degrading enzymes, resulting in superior therapeutic activity to hyaluronic acid. In the study by Kariya (Kariya H, Yoshihara Y, Nakao Y, Sakurai N, Ueno M, Hashimoto M, Suda Y. Carboxymethyl-chitin promotes chondrogenesis by inducing the production of growth factors from immune cells. J Biomed Mater Res A. 2010 Sep 15; 94 (4): 1034-41), carboxymethyl chitin is co-cultured with peritoneal exudate cells *in vitro* to stimulate the production of transforming growth factor-β1 (TGF-β1) by the peritoneal exudate cells, and a resulting culture supernatant is then co-cultured with chondrocytes to induce the RNA expression of chondrogenic transcription factor Sox9, matrix proteoglycans, Col2α1, and Comp, thereby promoting chondrogenesis. In the study by Wu Hongbin et al. (Hongbin W, Jingyuan D, Linyun C, Yuming D. Carboxymethylated chitin reduces MMP-1 expression in rabbit ACLT osteoarthritic cartilage. Ann Rheum Dis. 2004 Apr; 63(4): 369-72), the injection of carboxymethyl chitin into an OA model can significantly reduce the mRNA expression level of matrix metalloproteinase 1 (MMP-1) in the osteoarthritic joint, contributing to alleviation of cartilage degeneration. It can be seen that carboxymethyl chitin exhibits excellent affinity for chondrocytes, and can promote the proliferation and matrix secretion of chondrocytes, thereby alleviating OA. Currently, there is a widespread demand for viscosupplements based on carboxymethyl chitin. Thus, the development of preparation processes for these viscosupplements holds significant significance.

The existing conventional processes for preparing viscosupplements based on carboxymethyl chitin require the treatment of gels through alcohol precipitation or similar procedures during production. As a result, unnecessary organic solvents (for example, ethanol) are introduced, which not only causes discomfort and pain after injection but also fails to meet the relevant regulatory requirements. Further, gels treated by alcohol precipitation or similar procedures have some drawbacks in structural stability and mechanical properties.

There remains an urgent need in the art for a novel method to produce a viscosupplement exhibiting high stability, favorable mechanical properties, and high safety without introducing unnecessary organic solvents.

### SUMMARY

To solve the above technical problems, the present application provides a method for preparing a viscosupplement, where the viscosupplement includes 0.5 wt% to 5 wt% of crosslinked carboxymethyl chitin and 0.5 wt% to 1.8 wt% of a buffer; the buffer is selected from the group consisting of a phosphate, a borate, a citrate, or any combination thereof; the viscosupplement has a pH of 7.0±0.5 and an osmotic pressure of 260 mOsm/kg to 400 mOsm/kg; the crosslinked carboxymethyl chitin includes carboxymethyl chitin and a crosslinking agent; the crosslinking agent includes two or more amino groups and is selected from the group consisting of a chitosan oligosaccharide, lysine, polylysine, arginine, polyarginine, cystamine, spermidine, spermine, putrescine and a salt thereof, polyethylene glycol diamine, carnosine, an antimicrobial peptide, a collagen derived peptide, a cell binding peptide, an Arg-Gly-Asp (RGD) peptide, an RGD derived peptide, or any combination thereof; a degree of modification (MoD) of the crosslinked carboxymethyl chitin is 10% to 18%; and the method includes:
a) dissolving the crosslinking agent to produce a crosslinking agent reaction solution, where the crosslinking agent reaction solution includes a buffer system with a pH of 5 to 9;
b) adding the carboxymethyl chitin to the crosslinking agent reaction solution, and mixing, where a molar ratio of carboxyl groups in the carboxymethyl chitin to amino groups in the crosslinking agent is 3:2 to 1:2;
c) adding a condensing agent to initiate an amidation reaction between the carboxymethyl chitin and the crosslinking agent, where a molar ratio of the carboxyl groups in the carboxymethyl chitin to the condensing agent is 4:1 to 1:2, and the condensing agent is selected from the group consisting of 1-ethyl-3-(3-dimethylaminopropyl)carbodiimide (EDC), 4-(4,6-dimethoxy-1,3,5-triazine-2-yl)-4-methyl-morpholinium chloride (DMTMM), 1-ethyl-3-(3-dimethylaminopropyl)carbodiimide hydrochloride (EDCI), 2-chloro-4,6-dimethoxy-1,3,5-triazine (CDMT), or a combination thereof;
d) diluting a resulting gel with the buffer to 0.5% to 5% (w/w); and
e) conducting dialysis with the buffer to produce the viscosupplement,
where the method does not include use of ethanol, acetone, or methanol.

### BEIRF DESCRIPTION OF THE DRAWINGS

The present application will be described in further detail below with reference to accompanying drawings.
FIG. 1 is a picture illustrating the stringiness of a polyethylene glycol diamine-crosslinked carboxymethyl chitin gel;
FIG. 2 shows a comparison of macroscopic characterizations of crosslinked carboxymethyl chitin gels prepared with and without alcohol precipitation;
FIG. 3 shows a comparison of microstructures of crosslinked carboxymethyl chitin gels prepared with and without alcohol precipitation;
FIG. 4 shows a surface of a rabbit cartilage joint in each group that is observed by scanning electron microscopy at week 19 after surgery;
FIG. 5 shows safranin O-fast green stained sections (10×) of cartilages from a group B and a group E at week 18 after surgery; and
FIG. 6 shows immunohistochemical staining results (10×) of type II collagen for a group B, a group D, and a group E at week 18 after surgery.

### DETAILED DESCRIPTION OF THE EMBODIMENTS

The present application relates to a method for preparing a viscosupplement. The viscosupplement includes 0.5 wt% to 5 wt% of crosslinked carboxymethyl chitin and 0.5 wt% to 1.8 wt% of a buffer. The buffer is selected from the group consisting of a phosphate, a borate, a citrate, or any combination thereof. The viscosupplement has a pH of 7.0±0.5 and an osmotic pressure of 260 mOsm/kg to 400 mOsm/kg. The crosslinked carboxymethyl chitin includes carboxymethyl chitin and a crosslinking agent. The crosslinking agent includes two or more amino groups and is selected from the group consisting of a chitosan oligosaccharide, lysine, polylysine, arginine, polyarginine, cystamine, spermidine, spermine, putrescine and a salt thereof, polyethylene glycol diamine, carnosine, an antimicrobial peptide, a collagen derived peptide, a cell binding peptide, an RGD peptide, an RGD derived peptide, or any combination thereof. MoD of the crosslinked carboxymethyl chitin is 10% to 18%. The method for preparing a viscosupplement includes the following step: a) The crosslinking agent is dissolved to produce a crosslinking agent reaction solution. The crosslinking agent reaction solution includes a buffer system with a pH of 5 to 9. In an embodiment, the viscosupplement has a pH of 7.2 to 7.4. In an embodiment, the crosslinking agent includes a non-toxic and biocompatible crosslinking agent. In an embodiment, the crosslinking agent includes an amino acid. In a preferred embodiment, the crosslinking agent is selected from the group consisting of lysine, polyethylene glycol diamine, and cystamine. Viscosupplements produced from different crosslinking agents under similar crosslinking conditions may exhibit varying rheological properties and degradation characteristics to some extent. In an embodiment, the buffer includes a borate. In an embodiment, the buffer includes a citrate. In an embodiment, the buffer includes a phosphate. In the present application, the term "MoD" refers to a percentage of carboxymethyl glucosamine monomers that are modified by the crosslinking agent in the crosslinked carboxymethyl chitin. In an embodiment, the crosslinked carboxymethyl chitin has MoD of 8% to 20%. In an embodiment, the crosslinked carboxymethyl chitin has MoD of 11% to 15%. In an embodiment, the crosslinked carboxymethyl chitin has MoD of 14% to 16%. In an embodiment, the crosslinked carboxymethyl chitin has MoD of about 15%. In an embodiment, the buffer system includes one or a combination of salts of a chloride ion, a borate ion, an acetate ion, a sulfate ion, a phosphate ion, a citrate ion, and a hydrogen phosphate ion. In an embodiment, the salts include alkali metal salts. In an embodiment, the salts include sodium salts or potassium salts. In an embodiment, the buffer system includes a phosphate ion. In an embodiment, the buffer system includes a citrate ion. In an embodiment, a total molar concentration of anions in the buffer system is 0.02 M to 2.0 M. In a preferred embodiment, the pH of the buffer system is 6 to 8. In a further preferred embodiment, the pH of the buffer system is 7.0±0.5. In an embodiment, a concentration of the crosslinking agent in the crosslinking agent reaction solution is 0.005 g/mL to 0.1 g/mL. In an embodiment, the concentration of the crosslinking agent in the crosslinking agent reaction solution is 0.01 g/mL to 0.05 g/mL. In an embodiment, the concentration of the crosslinking agent in the crosslinking agent reaction solution is about 0.01 g/mL.

The method for preparing a viscosupplement further includes the following step: b) The carboxymethyl chitin is mixed with the crosslinking agent reaction solution. A molar ratio of carboxyl groups in the carboxymethyl chitin to amino groups in the crosslinking agent is 3:2 to 1:2. In an embodiment, the carboxymethyl chitin has a degree of deacetylation of no more than 20%, a degree of carboxymethyl substitution of 1.0 to 2.0, a molecular weight of no less than 300 kDa, and a dynamic viscosity of no less than 4,000 mPa·s (at 25 mg/mL). In an embodiment, the degree of deacetylation of the carboxymethyl chitin is 10% to 18%. In an embodiment, the degree of deacetylation of the carboxymethyl chitin is 12% to 15%. In an embodiment, the degree of deacetylation of the carboxymethyl chitin is about 13.7%. In an embodiment, the degree of carboxymethyl substitution of the carboxymethyl chitin is 1.1 to 1.5. In an embodiment, the degree of carboxymethyl substitution of the carboxymethyl chitin is about 1.28. In an embodiment, the dynamic viscosity of the carboxymethyl chitin is 5,000 mPa·s to 6,000 mPa·s (at 25 mg/mL). In an embodiment, the dynamic viscosity of the carboxymethyl chitin is about 5,174 mPa·s (at 25 mg/mL). In an embodiment, a weight-average molecular weight of the carboxymethyl chitin is 1 * 10⁵ g/mol to 1 * 10⁷ g/mol. In an embodiment, the weight-average molecular weight of the carboxymethyl chitin is 5 * 10⁵ g/mol to 1 * 10⁶ g/mol. In an embodiment, the weight-average molecular weight of the carboxymethyl chitin is about 7.11 * 10⁵ g/mol. In an embodiment, substitutions at a position C6 account for 50% to 80% of total substitutions, substitutions at a position C3 account for 10% to 20% of the total substitutions, and substitutions at an amino position account for 10% to 30% of the total substitutions. In a preferred embodiment, the substitutions at the position C6 account for about 70% of the total substitutions, the substitutions at the position C3 account for about 10% of the total substitutions, and the substitutions at the amino position account for about 20% of the total substitutions. In an embodiment, a mass concentration of the carboxymethyl chitin is 1% to 10% (w/w). In a preferred embodiment, the mass concentration of the carboxymethyl chitin is 2.5% to 6% (w/w). In an embodiment, the molar ratio of the carboxyl groups in the carboxymethyl chitin to the amino groups in the crosslinking agent is 3:2 to 1:1. In an embodiment, the molar ratio of the carboxyl groups in the carboxymethyl chitin to the amino groups in the crosslinking agent is about 9:7. In an embodiment, in the step b), a temperature is 10±5°C.

The method for preparing a viscosupplement further includes the following step: c) A condensing agent is added to initiate an amidation reaction between the carboxymethyl chitin and the crosslinking agent. A molar ratio of the carboxyl groups in the carboxymethyl chitin to the condensing agent is 4:1 to 1:2. The condensing agent is selected from the group consisting of EDC, DMTMM, EDCI, CDMT, or a combination thereof. In an embodiment, the condensing agent is an aqueous solution. In an embodiment, a concentration of the condensing agent is 2% to 20% (w/w). In an embodiment, the molar ratio of the carboxyl groups in the carboxymethyl chitin to the condensing agent is 3:2 to 1:1. In an embodiment, the molar ratio of the carboxyl groups in the carboxymethyl chitin to the condensing agent is about 9:7. In an embodiment, in the step c), a reaction time is 24 h to 96 h. In a preferred embodiment, in the step c), the reaction time is 48 h to 72 h. In an embodiment, in the step c), a temperature is 10±5°C.

The method for preparing a viscosupplement further includes the following step: d) A resulting gel is diluted with a buffer to 0.5% to 5% (w/w). In an embodiment, the gel is diluted to a concentration of 1.5% to 4% (w/w). In an embodiment, the gel is diluted to a concentration of about 4% (w/w). In an embodiment, the gel is diluted to a concentration of about 2.0% (w/w). In an embodiment, the gel is diluted to a concentration of about 1.5% (w/w).In an embodiment, the buffer is selected from the group consisting of a phosphate, a borate, a citrate, or any combination thereof. In an embodiment, the buffer includes a borate. In an embodiment, the buffer includes a citrate. In an embodiment, a pH of the buffer is 7.0±0.5. In a preferred embodiment, the pH of the buffer is 7.2 to 7.4.

The method for preparing a viscosupplement further includes the following step: e) Dialysis is conducted with a buffer to produce the viscosupplement. In an embodiment, the dialysis is conducted to a concentration of 1.0% to 5.4% (w/w). In an embodiment, the dialysis is conducted for 72 h to 180 h. In an embodiment, the buffer is selected from the group consisting of a phosphate, a borate, a citrate, or any combination thereof. In an embodiment, the buffer includes a borate. In an embodiment, the buffer includes a citrate. In an embodiment, the buffer used for the dialysis is the same as the buffer used for the diluting. In an embodiment, a pH of the buffer is 7.0±0.5. In a preferred embodiment, the pH of the buffer is 7.2 to 7.4. In an embodiment, the buffer used for the dialysis is the same as the buffer system used in the step a). In an embodiment, a volume/weight of the buffer is 5 times to 20 times a volume/weight of the gel. In an embodiment, the volume/weight of the buffer is about 10 times the volume/weight of the gel.

The method for preparing a viscosupplement does not involve ethanol, acetone, or methanol. In an embodiment, the method excludes the use of an organic solvent, and the organic solvent may have a similar function to the ethanol, acetone, or methanol. In an embodiment, the method excludes washing or precipitation with ethanol, acetone, or methanol. In an embodiment, the method excludes washing or precipitation with an organic solvent, for example, alcohol precipitation or ethanol precipitation.

Optionally, the method for preparing a viscosupplement can further include the following step: f) The viscosupplement obtained in the step e) is pre-filled into a glass syringe and subjected to high-temperature sterilization. In an embodiment, the pre-filling includes pre-filled sealing. In an embodiment, a filling volume is 1 mL to 10 mL. In an embodiment, the filling volume is 1 mL, 1.1 mL, 1.2 mL, 1.3 mL, 1.4 mL, 1.5 mL, 1.6 mL, 1.7 mL, 1.8 mL, 1.9 mL, 2 mL, 2.1 mL, 2.2 mL, 2.3 mL, 2.4 mL, 2.5 mL, 2.6 mL, 2.7 mL, 2.8 mL, 2.9 mL, 3 mL, 3.1 mL, 3.2 mL, 3.3 mL, 3.4 mL, 3.5 mL, 3.6 mL, 3.7 mL, 3.8 mL, 3.9 mL, 4 mL, 4.1 mL, 4.2 mL, 4.3 mL, 4.4 mL, 4.5 mL, 4.6 mL, 4.7 mL, 4.8 mL, 4.9 mL, 5 mL, 5.1 mL, 5.2 mL, 5.3 mL, 5.4 mL, 5.5 mL, 5.6 mL, 5.7 mL, 5.8 mL, 5.9 mL, 6 mL, 6.1 mL, 6.2 mL, 6.3 mL, 6.4 mL, 6.5 mL, 6.6 mL, 6.7 mL, 6.8 mL, 6.9 mL, 7 mL, 7.1 mL, 7.2 mL, 7.3 mL, 7.4 mL, 7.5 mL, 7.6 mL, 7.7 mL, 7.8 mL, 7.9 mL, 8 mL, 8.1 mL, 8.2 mL, 8.3 mL, 8.4 mL, 8.5 mL, 8.6 mL, 8.7 mL, 8.8 mL, 8.9 mL, 9 mL, 9.1 mL, 9.2 mL, 9.3 mL, 9.4 mL, 9.5 mL, 9.6 mL, 9.7 mL, 9.8 mL, 9.9 mL, or 10 mL. In an embodiment, the high-temperature sterilization includes moist-heat sterilization. In an embodiment, the high-temperature sterilization is conducted at 110°C to 130°C. In an embodiment, the high-temperature sterilization is conducted at about 121°C. In an embodiment, the high-temperature sterilization is conducted for no less than 15 min. In an embodiment, the high-temperature sterilization is conducted for about 15 min. In an embodiment, an F0 value is not less than 12. In an embodiment, a dynamic viscosity of the viscosupplement after the high-temperature sterilization is 60% to 118% of a dynamic viscosity of the viscosupplement before the high-temperature sterilization.

The present application may also relate to a viscosupplement prepared by a method for preparing the viscosupplement. In an embodiment, the method for preparing the viscosupplement may include the method for preparing a viscosupplement according to any one of the above embodiments. In an embodiment, the viscosupplement may include the viscosupplement according to any one of the above embodiments. In an embodiment, the viscosupplement has a long duration of action. In an embodiment, a degradation time of the viscosupplement is not less than 4 weeks. In an embodiment, the viscosupplement is a homogeneous gel. In an embodiment, the viscosupplement has a dynamic viscosity of no less than 1,000 mPa·s at a shear rate of 10 s⁻¹ under the condition of 25±2°C. In an embodiment, under the condition of 25±2°C, the viscosupplement has a storage modulus of 3 Pa to 40 Pa and a loss modulus of 5 Pa to 13 Pa at 2.5 Hz and has a storage modulus of 10 Pa to 100 Pa and a loss modulus of 10 Pa to 25 Pa at 10 Hz. In an embodiment, the storage modulus and loss modulus of the viscosupplement increase with the increase in shear frequency during motion. In an embodiment, the viscosupplement exhibits excellent biocompatibility, has no toxicity itself and/or does not lead to a toxic degradation product, does not cause an inflammatory response, and does not exacerbate OA inflammation. In an embodiment, the viscosupplement is readily injectable. In an embodiment, the viscosupplement can be injected through an 18G, 19G, 20G, 21G, 22G, 23G, 24G, 25G, 26G, or 27G needle. In an embodiment, the viscosupplement can be injected into a joint cavity. In an embodiment, an administration frequency for the viscosupplement is less than once per week. In an embodiment, the administration frequency for the viscosupplement is less than once per two weeks. In a preferred embodiment, the administration frequency for the viscosupplement is about once per month. In an embodiment, a dosage of the viscosupplement is determined based on a size of a joint cavity. In an embodiment, the dosage of the viscosupplement is 1 mL to 5 mL per joint. In an embodiment, three administrations of the viscosupplement constitute one course of treatment.

Compared with the prior art, the present disclosure has the following beneficial effects:
1. The method for preparing a viscosupplement in the present disclosure does not involve alcohol precipitation that introduces an unnecessary organic solvent, which avoids post-injection discomfort and pain and also complies with relevant regulatory requirements.
2. In terms of gel transparency, uniformity of microporous structures, and structural stability, the viscosupplement prepared by the method of the present disclosure is significantly better than a viscosupplement prepared based on alcohol precipitation.
3. Compared with a viscosupplement prepared based on alcohol precipitation, the viscosupplement prepared by the method of the present disclosure exhibits significantly improved cohesiveness, and can well recover its original properties after deformation under shear stress, thereby maintaining the protective effect on articular cartilage.
4. The viscosupplement prepared by the present disclosure exhibits excellent thermal stability, and can undergo moist-heat sterilization, enabling a high degree of sterility assurance and improved safety.
5. The present disclosure employs a natural amino acid or an amino-rich natural substance as a crosslinking agent. Such a crosslinking agent is non-toxic. The crosslinked carboxymethyl chitin and a degradation product thereof exhibit prominent biocompatibility.
6. The viscosupplement prepared by the present disclosure is a homogeneous gel with excellent flexibility, no granular sensation, low gel swelling, suitable rheological properties, and a moderate elastic modulus. Consequently, the viscosupplement can reduce tissue compression caused by increased swelling volume or excessive elastic modulus while providing protection.
7. The viscosupplement prepared by the present disclosure exhibits excellent compatibility with cartilage, and can promote the proliferation of chondrocytes and alleviate the symptoms of OA.
8. The viscosupplement prepared by the present disclosure has a low friction coefficient, and a storage modulus and a loss modulus that increase with shear during motion. Thus, the viscosupplement can effectively resist compression during joint motion and provide excellent lubricating performance.
9. When the viscosupplement prepared by the present disclosure is degraded by lysozyme, there is a long degradation time and a degradation product with a large molecular weight, indicating the resistance of the viscosupplement to enzymatic degradation.
10. The viscosupplement prepared by the present disclosure has a specifically selected osmotic pressure, which avoids pain caused by osmotic imbalance (either excessively high or excessively low osmotic pressure).
11. In the viscosupplement prepared by the present disclosure, an appropriate molar ratio of carboxyl groups to amino groups in the crosslinking agent is selected to affect a reaction degree and MoD. If the molar ratio of the carboxyl groups to the amino groups in the crosslinking agent is too large, the viscosupplement has excessively low MoD and excessive swelling, which easily causes water absorption-induced swelling after injection. If the molar ratio of the carboxyl groups to the amino groups in the crosslinking agent is too small, the viscosupplement has excessively high MoD and presents a granular state.
12. In the present disclosure, an appropriate degree of deacetylation and an appropriate degree of substitution are selected to guarantee the solubility, biocompatibility, a suitable molecular weight, and rheological properties of carboxymethyl chitin, which contributes to the stability of mechanical properties of a crosslinked product. When the degree of deacetylation exceeds 50%, the material is considered chitosan, which suffers from solubility issues. In addition, when the degree of carboxymethyl substitution increases, the equilibrium swelling degree and pH swelling sensitivity of chitin increase, and the cation adsorption capacity is also enhanced. The degree of substitution at the position C6 (to enhance water solubility), the degree of substitution at the position C3 (to impart specified resistance to lysozyme degradation), and the degree of substitution at the amino position (to facilitate the adjustment of charge properties of a side chain) are specifically selected to balance the biocompatibility and antibacterial activity of chitin.
13. The viscosupplement prepared by the present disclosure exhibits high safety and has an effect of alleviating OA pain.
14. The carboxymethyl chitin obtained through process control has the following advantages: The solubility of chitin is improved, such that chitin insoluble in acids, alkalis, and neutral solutions becomes a water-soluble material. In addition, deacetylation is not required, which can prevent the exposure of cationic amino groups that may cause inflammatory responses.

### Examples

The present application will be described in detail below through exemplary specific examples. The following examples are only intended to help those skilled in the art better understand the various inventions of the present application. It should be noted that the spirit of the present application and the scope of protection of the claims are not limited by the following specific examples.

### List of reagents and devices

| Reagent/device name | Catalog No./Item No. | Manufacturer |
|---|---|---|
| Dialysis bag | / | Shanghai Zhenjian Biotechnology Co., Ltd. |
| Glass syringe | / | BD |
| HAAKE MARS III rheometer | / | HAAKE MARS |
| Lysozyme | CAS: 12650-88-3 | Shanghai Yuanju Bio-tech Co., Ltd. |
| Constant-temperature forced-air oven | DHG-9245A | Shanghai Huitai Instrument Manufacturing Co., Ltd. |
| Size exclusion chromatography | DAWN & Optilab Trex | WYATT |
| 1 mL syringe | / | BD |
| 18G needle | / | Weigao Group |
| Universal testing machine | AI-3000 | GOTECH |
| Scanning electron microscope | TM4000 | HITACHI |
| HPLC-ELSD | 1260 Infinity | Agilent |
| C18 silica bonded phase chromatography column | TC-C18 | Agilent |

Unless otherwise specified, all other reagents and devices are conventional in the art or commercially available.

### Example 1 Polyethylene glycol diamine-crosslinked carboxymethyl chitin gel and preparation thereof

4 g of polyethylene glycol diamine (average molecular weight: 400, Sigma) was weighed and uniformly dissolved in 100 mL of a citrate buffer (which was prepared by dissolving 7.9 g of sodium citrate dihydrate and 1.3 g of citric acid monohydrate in 1,000 g of water for injection). Then, 5 g of carboxymethyl chitin (Haoleyuan Biotechnology Co., Ltd., degree of deacetylation: 13.7%; transmittance of 1 mg/L solution: 100.02%; pH of 30 mg/mL solution: 7.31; dynamic viscosity at 25 mg/mL: 5,174 mPa·s (shear rate: 10 s⁻¹); weight-average molecular weight: 7.11 * 10⁵ g/mol; degree of substitution: 1.28; isoelectric point: 2.6; and endotoxin level: less than 0.025 EU/mg, the same below) was added, and stirring was conducted at 300 rpm for 10 h to produce a homogeneous reaction solution. 14 mL of an aqueous solution including 2.7 g of EDC (Sigma) and 2 g of N-hydroxysuccinimide (NHS) (Sigma) was added to the reaction solution, thorough mixing was allowed, and a reaction was conducted at 15°C for 24 h. A crosslinked carboxymethyl chitin gel was diluted with a neutral citrate buffer (which was prepared by dissolving 9.1 g of sodium citrate dihydrate and 0.5 g of citric acid monohydrate in 1,000 g of water for injection and then adjusting to a pH of 7.2) to 4% (w/w). Dialysis was conducted with the neutral citrate buffer at 15°C for 180 h with buffer replacement once every 24 h. A volume of an external dialysis buffer was 10 times a volume of the crosslinked carboxymethyl chitin gel. The polyethylene glycol diamine-crosslinked carboxymethyl chitin gel was filled into 3 mL BD glass syringes and subjected to moist-heat sterilization at 121°C for 15 min to produce a polyethylene glycol diamine-crosslinked carboxymethyl chitin viscosupplement. MoD of the polyethylene glycol diamine-crosslinked carboxymethyl chitin viscosupplement was determined according to the following specific process:

2 g of the polyethylene glycol diamine-crosslinked carboxymethyl chitin gel was added to 10 mL of an acetic acid-sodium acetate buffer including 1 mg/mL of lysozyme (18 g of sodium acetate was taken, 9.8 mL of glacial acetic acid was added, and water was added to 1,000 mL to produce the acetic acid-sodium acetate buffer) (activity unit: higher than or equal to 20,000 U/mg). Degradation was allowed in a constant-temperature forced-air oven at 55°C for 72 h. 1 mL of a resulting reaction solution was taken, diluted with 50 mL of a methanol/water mobile phase solution (methanol/water ratio = 80:20), filtered through a 0.45 µm filter, and then analyzed for PEG content through gradient elution by high performance liquid chromatography-evaporative light scattering detector (HPLC-ELSD). Detection conditions were as follows: C18 silica bonded phase chromatography column; flow rate: 1.0 mL/min; column temperature: 25°C; and injection volume: 20 µL.

A content of N-acetylglucosamine after hydrochloric acid hydrolysis of carboxymethyl chitin was determined by a 3,5-dinitrosalicylic acid (DNS) method. 0.2 g of the crosslinked carboxymethyl chitin gel was weighed, and 2.0 mL of a 5 M HCl solution was added for acid hydrolysis. A resulting mixture was sealed in an ampoule bottle, and the ampoule bottle was placed in a 105°C forced-air oven for 30 min and then taken out. After the ampoule bottle was cooled, 2 mL of a 5 M NaOH solution was added to adjust a pH to 7 to 9, and dilution was conducted to a volume of 5 mL. 600 mL of a DNS test solution (91 g of potassium sodium tartrate was added to 500 mL of water, and stirring was conducted at 50°C for dissolution. 3.15 g of DNS and 20 g of NaOH were then added successively. 2.5 g of crystalline phenol and 2.5 g of anhydrous sodium sulfite were added, and stirring was conducted for dissolution. Cooling was allowed, and water was added to a volume of 1,000 mL) was added, and thorough mixing was conducted. Boiling was conducted in a boiling water bath for 5 min, and then cooling was conducted to room temperature. Absorbance values of sample and glucosamine hydrochloride standard solutions at 520 nm were measured and compared to determine a sugar unit content in the crosslinked carboxymethyl chitin. MoD was calculated.

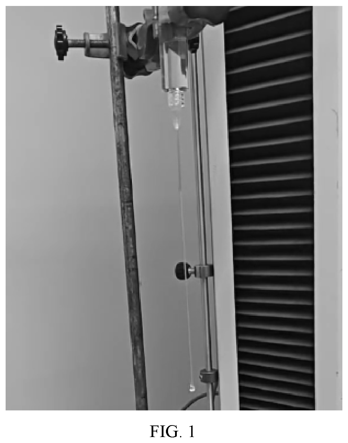

Average MoD was determined to be 17.31%. The gel exhibits excellent cohesiveness and stringiness (as shown in FIG. 1).

### Example 2 Amino acid-crosslinked carboxymethyl chitin gel and preparation thereof

1 g of lysine (Sinopharm Chemical Reagent Co., Ltd.) was weighed and dissolved in 100 mL of a phosphate buffer (which was prepared from 8.5% of sodium chloride, 0.5% of disodium hydrogen phosphate, 0.15% of sodium dihydrogen phosphate, and water for injection, and had a pH of 7.2). After complete dissolution, 4.0 g of carboxymethyl chitin (Haoleyuan Biotechnology Co., Ltd.) was added, and stirring was conducted at 200 rpm for 6 h to produce a homogeneous reaction solution. 12 mL of an aqueous solution including 4 g of DMTMM (Shanghai Dibai Biotechnology Co., Ltd.) was added to the reaction solution, thorough mixing was allowed, and a reaction was conducted at 8°C for 72 h. A crosslinked carboxymethyl chitin gel was diluted with the phosphate buffer to 2.0% (w/w). Dialysis was conducted with the phosphate buffer at 8°C for 120 h with buffer replacement once every 24 h. A volume of an external dialysis buffer was 10 times a volume of the crosslinked carboxymethyl chitin gel. The crosslinked carboxymethyl chitin gel was filled into 2 mL BD glass syringes and subjected to moist-heat sterilization at 121°C for 15 min to produce the amino acid-crosslinked carboxymethyl chitin viscosupplement. MoD of the amino acid-crosslinked carboxymethyl chitin viscosupplement was detected by the method described in Example 1. Specifically, a lysine content after enzymatic hydrolysis of the crosslinked carboxymethyl chitin was detected using an amino acid analyzer, and the lysine content was then divided by a sugar unit content to obtain MoD. Average MoD was determined to be 15.31%.

### Example 3 Cystamine-crosslinked carboxymethyl chitin gel and preparation thereof

1.5 g of cystamine (Beijing J&K Scientific) was weighed and dissolved in 100 mL of a phosphate buffer (which was prepared from 8.5% of sodium chloride, 0.5% of disodium hydrogen phosphate, 0.15% of sodium dihydrogen phosphate, and water for injection, and had a pH of 7.2). After complete dissolution, 3.0 g of carboxymethyl chitin (Haoleyuan Biotechnology Co., Ltd.) was added, and stirring was conducted at 100 rpm for 12 h to produce a homogeneous reaction solution. 10 mL of an aqueous solution including 2.5 g of DMTMM (Shanghai Dibai Biotechnology Co., Ltd.) was added to the reaction solution, thorough mixing was allowed, and a reaction was conducted at 6°C for 48 h. A crosslinked carboxymethyl chitin gel was diluted with a borate buffer (which was prepared by dissolving 8.5 g of sodium chloride, 1.9 g of borax (decahydrate), and 11.1 g of boric acid in 1,000 g of water for injection and adjusting to a pH of 7.4) to 1.5% (w/w). Dialysis was conducted with the borate buffer at 6°C for 72 h with buffer replacement twice every 24 h. A volume of an external dialysis buffer was 10 times a volume of the crosslinked carboxymethyl chitin gel. The crosslinked carboxymethyl chitin gel was filled into 1 mL BD glass syringes and subjected to moist-heat sterilization at 121°C for 15 min to produce the cystamine-crosslinked carboxymethyl chitin viscosupplement. MoD of the cystamine-crosslinked carboxymethyl chitin viscosupplement was detected by the method described in Example 1. A sulfur (S) element content was detected using an elemental analyzer. A ratio of half the sulfur element content to a sugar unit content was then calculated to obtain MoD. Average MoD was determined to be 11.2%.

### Example 4 Crosslinked carboxymethyl chitin gels with different MoDs and comparison of properties of the crosslinked carboxymethyl chitin gels

With cystamine as a crosslinking agent, crosslinked carboxymethyl chitin materials with different MoDs were prepared by the method described in Example 3. Data was shown in Table 1:

**Table 1**

| Crosslinki ng agent concentrati on/% | Carboxym ethyl chitin concentrati on/% | Condensin g agent concentrati on/% | Pre-sterilizat ion dynamic viscosity/mP a·s | Post-sterilizat ion dynamic viscosity/mPa ·s | Average MoD | Post-sterilizati on status |
|---|---|---|---|---|---|---|
| 0.455 | 2.727 | 2.273 | Dilute, undetectable | Dilute, undetectable | 4.23% | Viscous solution |
| 0.909 | 2.727 | 2.273 | 2266 | Dilute, undetectable | 7.88% | Viscous solution |
| 1.36 | 2.727 | 2.273 | 5823 | 4234 | 11.20% | Gel |
| 1.818 | 2.727 | 2.273 | 5430 | 3678 | 14.60% | Gel |
| 2.273 | 2.727 | 2.273 | 3156 | 2137 | 21.30% | Granulated gel, phase separation |

According to the data in Table 1, MoD of 8% to 20% is currently considered suitable for the material. When the MoD of the material is lower than 8%, the material swells excessively and cannot withstand moist heat sterilization. When the MoD of the material exceeds 20%, the material presents a granular state and no longer presents a homogeneous state.

### Example 5 Stability of crosslinked carboxymethyl chitin gels before and after sterilization

Gels obtained both before and after sterilization in Examples 1, 2, and 3, a non-crosslinked carboxymethyl chitin viscosupplement (Libao Xi) before and after sterilization, and gels stored at (5±3)°C for 6 months after sterilization were each directly loaded onto a bottom plate of an HAAKE MARS III rheometer at (25±0.1)°C, and tested by a flow sweep at a shear rate of 1 s⁻¹ to 100 s⁻¹ using a cone plate (angle: 1°) according to an operating procedure of the rheometer. A dynamic viscosity at a shear rate of 10 s⁻¹ was selected for comparison. Data was shown in Table 2.

**Table 2 Dynamic viscosity changes of crosslinked carboxymethyl chitin gels before and after sterilization and gels stored for 6 months after sterilization**

| Compared samples | Pre-steriliz ation dynamic viscosity/mPa·s | Post-steriliz ation dynamic viscosity/mPa·s | Post-sterilization dynamic viscosity/pre-steril ization dynamic viscosity, % | Post-steriliz ation dynamic viscosity after 6 months of storage, mPa s | Post-sterilization dynamic viscosity after 6 months of storage/pre-sterili zation dynamic viscosity, % |
|---|---|---|---|---|---|
| Non-crossli nked carboxymet hyl chitin (15 mg/mL) | 658 | 214 | 32.5 | 198 | 30.1 |
| Example 1 (11.5 mg/mL) | 5266 | 6193 | 117.6 | 5883 | 111.7 |
| Example 2 (14.5 mg/mL) | 5218 | 5068 | 97.1 | 4997 | 95.8 |
| Example 3 (15 mg/mL) | 6103 | 4184 | 68.6 | 4356 | 71.4 |

According to the data in Table 2, the dynamic viscosity of the crosslinked carboxymethyl chitin gel is significantly higher than the dynamic viscosity of the non-crosslinked carboxymethyl chitin. Moreover, under the same sterilization conditions, the dynamic viscosity of the crosslinked carboxymethyl chitin gel exhibits a limited change rate, and consistently remains within the range of 60% to 118%. It can be seen that crosslinking can stabilize the structure of carboxymethyl chitin and enhance the thermal stability of carboxymethyl chitin, thereby enabling resistance to moist heat sterilization. In contrast, the dynamic viscosity of the non-crosslinked carboxymethyl chitin (the previous-generation product, Libao Xi) is greatly affected by moist heat sterilization, with a change rate exceeding 50%. This is because the structure of the non-crosslinked carboxymethyl chitin is disrupted at high temperatures. Thus, the non-crosslinked carboxymethyl chitin cannot undergo moist heat sterilization. Currently, the non-crosslinked carboxymethyl chitin is produced by aseptic processing. The terminal sterilization provides a significantly higher level of sterility assurance than the aseptic processing. Therefore, crosslinking not only improves the performance of the carboxymethyl chitin gel but also enhances the safety of the carboxymethyl chitin gel as an injection.

### Example 6 Characterization of crosslinked carboxymethyl chitin gels prepared with and without alcohol precipitation

### A. Crosslinked carboxymethyl chitin gel prepared with alcohol precipitation

1 g of lysine (Sinopharm Chemical Reagent Co., Ltd.) was weighed and dissolved in 100 mL of a phosphate buffer (which was prepared from 8.5% of sodium chloride, 0.5% of disodium hydrogen phosphate, 0.15% of sodium dihydrogen phosphate, and water for injection, and had a pH of 7.2). After complete dissolution, 4.0 g of carboxymethyl chitin (Haoleyuan Biotechnology Co., Ltd.) was added, and stirring was conducted at 200 rpm for 6 h to produce a homogeneous reaction solution. 12 mL of an aqueous solution including 4 g of DMTMM (Shanghai Dibai Biotechnology Co., Ltd.) was added to the reaction solution, thorough mixing was allowed, and a reaction was conducted at 8°C for 72 h. 95% ethanol was added in a volume 3 times a volume of a resulting reaction solution dropwise to the reaction solution under stirring. A resulting precipitate was collected and re-dissolved with a phosphate buffer to produce a crosslinked carboxymethyl chitin gel with a final concentration of 2.0% (w/w) (the precipitate was washed with ethanol 4 times, 6 times, or 8 times).

### B. Crosslinked carboxymethyl chitin gel prepared without alcohol precipitation

1 g of lysine (Sinopharm Chemical Reagent Co., Ltd.) was weighed and dissolved in 100 mL of a phosphate buffer (which was prepared from 8.5% of sodium chloride, 0.5% of disodium hydrogen phosphate, 0.15% of sodium dihydrogen phosphate, and water for injection, and had a pH of 7.2). After complete dissolution, 4.0 g of carboxymethyl chitin (Haoleyuan Biotechnology Co., Ltd.) was added, and stirring was conducted at 200 rpm for 6 h to produce a homogeneous reaction solution. 12 mL of an aqueous solution including 4 g of DMTMM (Shanghai Dibai Biotechnology Co., Ltd.) was added to the reaction solution, thorough mixing was allowed, and a reaction was conducted at 8°C for 72 h. A crosslinked carboxymethyl chitin gel was diluted with the phosphate buffer to 2.0% (w/w). Dialysis was conducted with the phosphate buffer at 8°C for 120 h with buffer replacement once every 24 h to produce a crosslinked carboxymethyl chitin gel with a final concentration of 2.0% (w/w). A volume of an external dialysis buffer was 10 times a volume of the crosslinked carboxymethyl chitin gel.

### C. Comparison

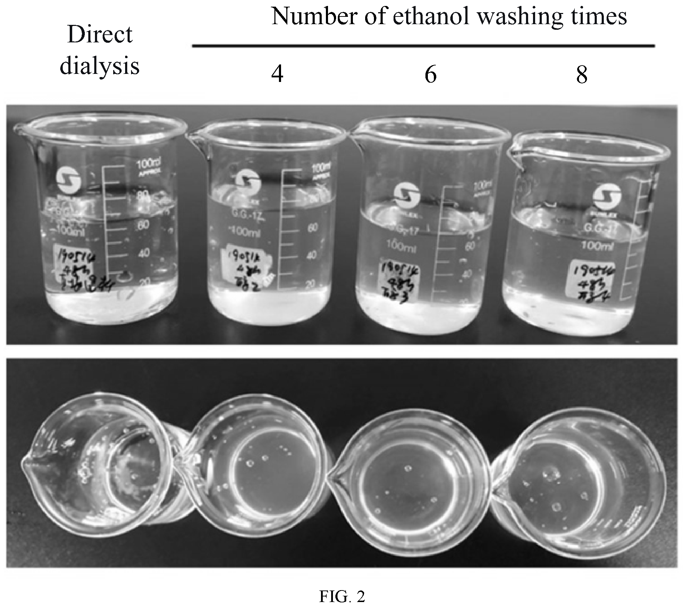

The crosslinked carboxymethyl chitin gel prepared without alcohol precipitation is different from the crosslinked carboxymethyl chitin gel prepared with alcohol precipitation in that, after the alcohol precipitation, a side-chain hydrogen bond structure of crosslinked carboxymethyl chitin is altered, and such an alteration readily leads to changes in the isoelectric point (an elevation in isoelectric point) and solubility (a decline in solubility) of carboxymethyl chitin, thereby resulting in changes in transparency and light transmittance. The most direct manifestation is turbidity. There is an isoelectric point change. Before crosslinking, the isoelectric point is 2.0 to 3.0. After crosslinking, the isoelectric point is 5.4 to 6.2. The gel produced after alcohol precipitation and re-dissolution has an isoelectric point changing slightly to 6.0 to 7.2. As shown in FIG. 2, there is a difference in appearance between the material prepared with alcohol precipitation and the material prepared without alcohol precipitation. In addition to the issue of ethanol residue, the alcohol precipitation leads to turbidity of the gel, which is detrimental to the lamp inspection after production (hindering the visual identification of impurities) and the control of product safety. Moreover, for tissue engineering medical devices, there are generally limits on ethanol residue because the presence of ethanol may inhibit some cellular activities.

Further analysis was conducted on the crosslinked carboxymethyl chitin prepared with alcohol precipitation (washing 4 times). Pre-sterilization and post-sterilization dynamic viscosities were detected according to the method described in Example 5. The pre-sterilization dynamic viscosity was 4,410 mPa·s, and the post-sterilization dynamic viscosity was 5,400 mPa·s. The post-sterilization dynamic viscosity was 122.4% of the pre-sterilization dynamic viscosity. The alcohol precipitation leads to dehydration of the crosslinked carboxymethyl chitin gel, which affects a hydration layer and a microstructure of the gel. During the subsequent re-dissolution process, the gel cannot return to its original structure, and the internal homogeneity of the gel is disrupted. Consequently, the gel cannot return its transparency. After being diluted several times, the material prepared with alcohol precipitation exhibits heterogeneity. Due to a more compact structure of the material achieved through alcohol precipitation, the dynamic viscosity of the material prepared with alcohol precipitation is reduced compared to the material prepared without alcohol precipitation. The post-sterilization dynamic viscosity exceeds 118% of the pre-sterilization dynamic viscosity, which is also related to this compact structure. In contrast, the crosslinked carboxymethyl chitin prepared without alcohol precipitation has a pre-sterilization dynamic viscosity of 5,218 mPa·s and a post-sterilization dynamic viscosity of 5,068 mPa·s, and the post-sterilization dynamic viscosity is 97.1% of the pre-sterilization dynamic viscosity (as shown in Table 2). A difference between the post-sterilization dynamic viscosity and the pre-sterilization dynamic viscosity of the crosslinked carboxymethyl chitin prepared without alcohol precipitation is significantly smaller than that of the crosslinked carboxymethyl chitin prepared with alcohol precipitation. It indicates that the crosslinked carboxymethyl chitin prepared without alcohol precipitation exhibits markedly superior structural stability during sterilization to the crosslinked carboxymethyl chitin prepared with alcohol precipitation.

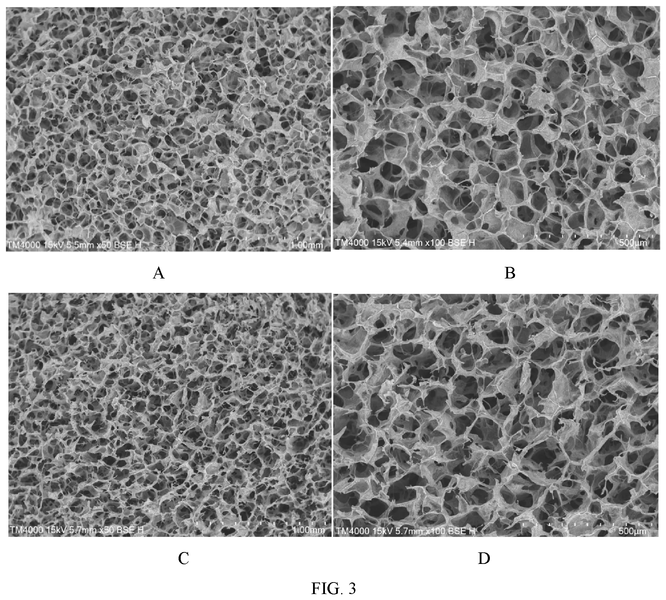

The crosslinked carboxymethyl chitin gels prepared with and without alcohol precipitation were lyophilized and then subjected to sputter coating. A microstructure of each lyophilized material was observed using a Hitachi tabletop microscope TM4000plus. The crosslinked carboxymethyl chitin gel prepared without alcohol precipitation has regular and uniform pores (as shown in FIG. 3: A at a magnification of 50 X and B at a magnification of 100 X). The crosslinked carboxymethyl chitin gel prepared with alcohol precipitation has more irregular pores than the crosslinked carboxymethyl chitin gel prepared without alcohol precipitation. This is because the alcohol precipitation disrupts the hydration of the material, leading to heterogeneity of the material. Consequently, the microstructure of the crosslinked carboxymethyl chitin gel prepared with alcohol precipitation also has pores of varying sizes (as shown in FIG. 3: C and D).

In summary, compared to the crosslinked carboxymethyl chitin gel prepared with alcohol precipitation, the crosslinked carboxymethyl chitin gel prepared without alcohol precipitation not only avoids ethanol residue but also has significant advantages in terms of gel transparency, uniformity of microporous structures, and structural stability, which further guarantees the performance and safety of the product.

### Example 7 Mechanical properties of crosslinked carboxymethyl chitin gels

Gels obtained after sterilization in Examples 1, 2, and 3 and a non-crosslinked carboxymethyl chitin viscosupplement (Libao Xi) were each directly loaded onto a bottom plate of an HAAKE MARS III rheometer at (25±0.1)°C, and tested for storage modulus and loss modulus at a frequency of 0.1 Hz to 100 Hz using a cone plate (angle: 1°) according to an operating procedure of the rheometer (strain range: 2%). Typically, a vibration frequency of the human body under normal conditions is approximately 2.5 Hz, and a vibration frequency of the human body under exercise conditions is approximately 10 Hz. The storage moduli and loss moduli at vibration frequencies of 2.5 Hz and 10 Hz were selected for comparison. Data was shown in Table 3.

**Table 3 Rheological properties of the crosslinked carboxymethyl chitin gels at the vibration frequencies of 2.5 Hz and 10 Hz**

| | Vibration frequency of 2.5 Hz | | Vibration frequency of 10 Hz | |
|---|---|---|---|---|
| | Storage modulus G' (Pa) | Loss modulus G" (Pa) | Storage modulus G' (Pa) | Loss modulus G" (Pa) |
| Non-crosslinked carboxymethyl chitin (15 mg/mL) | 1.023 | 2.321 | 1.879 | 3.564 |
| Example 1 (11.5 mg/mL) | 3.437 | 5.493 | 11.09 | 12.29 |
| Example 2 (14.5 mg/mL) | 8.573 | 7.913 | 16.34 | 16.13 |
| Example 3 (15 mg/mL) | 37.26 | 12.41 | 49.84 | 20.26 |

According to the data in Table 3, the crosslinked carboxymethyl chitin gels exhibit better viscoelastic properties than the non-crosslinked carboxymethyl chitin gel. The vibration frequencies of 2.5 Hz and 10 Hz can effectively simulate the motion conditions commonly encountered in joints. Typically, the synovial fluid in joints behaves as a liquid at low vibration frequencies. During motion, as the shear increases, the viscosity decreases. However, when the shear reaches a critical value, the synovial fluid transitions from a non-Newtonian fluid to a Newtonian fluid, resulting in pseudoplasticity and thixotropy to protect the articular cartilage. In this case, a storage modulus G' of the synovial fluid is usually greater than a loss modulus G" of the synovial fluid, and the storage modulus G' generally exceeds 10 Pa. The crosslinked carboxymethyl chitin gels have a storage modulus of 3 Pa to 40 Pa and a loss modulus of 5 Pa to 13 Pa at 2.5 Hz and have a storage modulus of 10 Pa to 100 Pa and a loss modulus of 10 Pa to 25 Pa at 10 Hz. Both the storage modulus and the loss modulus increase with the motion frequency. The crosslinked carboxymethyl chitin gels exhibit similar properties to the synovial fluid, and can effectively withstand the pressure during joint motion to protect damaged cartilage.

### Example 8 Degradation properties of crosslinked carboxymethyl chitin gels

20 g of each of the sterilized gels obtained in Examples 1, 2, and 3 and a non-crosslinked carboxymethyl chitin viscosupplement (Libao Xi) was weighed, 20 mL of an acetic acid-sodium acetate buffer including 1 mg/mL of lysozyme (18 g of sodium acetate was taken, 9.8 mL of glacial acetic acid was added, and water was added to 1,000 mL to produce the acetic acid-sodium acetate buffer) (activity unit: higher than or equal to 20,000 U/mg) was added, and degradation was conducted in a constant-temperature forced-air oven at 55°C for 48 h. An enzymatic degradation solution was subjected to precipitation with ethanol, and a resulting precipitate was vacuum-dried and re-dissolved with an acetic acid-sodium acetate buffer to produce a 6 mg/mL degradation product solution. The 6 mg/mL degradation product solution was filtered through a 0.2 µm filter and then tested for weight-average molecular weight by size exclusion chromatography. Each data point was measured in duplicate. Comparative data was shown in Table 4.

**Table 4 Weight-average molecular weights of in vitro degradation products of crosslinked carboxymethyl chitin gels**

| | Weight-average molecular weight after *in vitro* degradation * 10⁴ (g/mol) | |
|---|---|---|
| Non-crosslinked carboxymethyl chitin (15 mg/mL) | 1.452 | 1.470 |
| Example 1 (11.5 mg/mL) | 2.209 | 2.253 |
| Example 2 (14.5 mg/mL) | 2.164 | 2.214 |
| Example 3 (15 mg/mL) | 2.122 | 2.109 |

Lysozyme is a specific enzyme for chitin. The enzymatic degradation resistance of a gel can be indirectly evaluated by measuring a weight-average molecular weight of a degradation product of the gel after *in vitro* degradation. After enzymatic degradation, a product with a low weight-average molecular weight indicates more thorough degradation than a product with a high weight-average molecular weight. According to the data in Table 4, carboxymethyl chitin crosslinked through an amidation reaction is protected by a crosslinked structure, which enhances the resistance to enzymatic degradation *in vitro.* Therefore, it can be reasonably inferred that the crosslinked carboxymethyl chitin has longer *in vivo* degradation time than the non-crosslinked carboxymethyl chitin.

### Example 9 Characterization of cohesiveness of crosslinked carboxymethyl chitin gels prepared with and without alcohol precipitation and a non-crosslinked carboxymethyl chitin viscosupplement

The gels prepared without alcohol precipitation in Examples 1 to 3, the gel prepared with alcohol precipitation (washing 4 times) in Example 6, and the non-crosslinked carboxymethyl chitin viscosupplement (Chitogel) were each dispensed into 1 mL syringes fitted with 18G needles. The syringes were placed on a universal testing machine. After a segment of a gel was extruded by pushing a plunger at a rate of 5.0 mm/min, 10 drops of the fallen gel were collected. An average drop weight was calculated. A gel with a high average drop weight exhibits strong cohesiveness. Results were shown in Table 5. According to data in Table 5, the gels prepared without alcohol precipitation in Examples 1 to 3 all have an average drop weight of greater than 20 mg/mL, indicating prominent cohesiveness. The gels prepared without alcohol precipitation all exhibit significantly superior cohesiveness to the non-crosslinked carboxymethyl chitin viscosupplement and the gel prepared with alcohol precipitation. The cohesiveness typically originates from the intermolecular attractive forces within a gel. The better the cohesiveness, the better the stability and mechanical strength. During the viscosupplementation in joint cavities, a gel with excellent cohesiveness can well recover its original properties after deformation under shear stress, thereby maintaining a protective effect on articular cartilage.

**Table 5**

| Sample | Non-crosslinked carboxymethyl chitin | Example 1 | Example 2 | Example 3 | Example 6 |
|---|---|---|---|---|---|
| Average drop weight (mg) | 14 | 31 | 25 | 23 | 19 |
| N= 10 | | | | | |

### Example 10 Broad-spectrum antibacterial activity of crosslinked carboxymethyl chitin

To test the antibacterial activity of carboxymethyl chitin, *Staphylococcus epidermidis* (ATCC 29887), *Staphylococcus aureus* (ATCC 6538), *Escherichia coli* (ATCC 25922), *Pseudomonas aeruginosa* (ATCC 9027), and *Candida albicans* (ATCC 10231) were selected and each diluted with sterile normal saline to 5,000 CFU/mL. The sterile carboxymethyl chitin prepared in Example 2 was dissolved in sterile water for injection to prepare a 2% carboxymethyl chitin solution. The 2% carboxymethyl chitin solution was then thoroughly mixed in a ratio of 1:1 with a double-concentration sterile fluid thioglycollate medium (Shanghai Bowei Biotechnology Co., Ltd., 58.6 g of a fluid thioglycollate medium powder was dissolved in 1,000 mL of distilled water, boiled for complete dissolution, filtered while hot, dispensed, and sterilized at 115°C for 30 min) and soybean casein digest medium (Shanghai Bowei Biotechnology Co., Ltd., 60 g of a soybean casein digest medium powder was dissolved in 1,000 mL of distilled water, boiled for complete dissolution, filtered while hot, dispensed, and sterilized at 121°C for 15 min). A resulting mixed solution was serially diluted two-fold to 1:2, 1:4, 1:8, 1:16, 1:32, 1:64, 1:128, 1:256, 1:512, and 1:1024. *Staphylococcus epidermidis, Staphylococcus aureus,* and *Escherichia coli* were each inoculated into fluid thioglycollate media with different concentrations at a microbial inoculum size of no more than 100 CFU per tube, and cultured at 30°C to 35°C for 24 h. *Pseudomonas aeruginosa* and *Candida albicans* were each inoculated into soybean casein digest media with different concentrations at a microbial inoculum size of no more than 100 CFU per tube, and cultured at 20°C to 25°C for 24 h. A clarity level of each medium was observed. Gram staining was conducted for media produced after culturing to assess microbial growth, thereby determining the antibacterial activity. Results were shown in Table 6. The crosslinked carboxymethyl chitin material exhibits prominent broad-spectrum antibacterial activity.

**Table 6 Broad-spectrum antibacterial activity of crosslinked carboxymethyl chitin**

| Strain | 0 | 1:2 | 1:4 | 1:8 | 1:16 | 1:32 | 1:64 | 1:128 | 1:256 | 1:512 | 1:1024 |
|---|---|---|---|---|---|---|---|---|---|---|---|
| *Staphylococcus epidermidis* | + | - | - | - | - | - | - | - | - | - | + |
| *Staphylococcus aureus* | + | - | - | - | - | - | - | - | - | + | + |
| *Escherichia coli* | + | - | - | - | - | - | - | - | + | + | + |
| *Pseudomonas* | + | - | - | - | - | - | - | + | + | + | + |
| *aeruginosa* | | | | | | | | | | | |
| *Candida albicans* | + | - | - | + | + | + | + | + | + | + | + |

### Example 11 Protection of crosslinked carboxymethyl chitin on articular cartilage in an OA rabbit model

Based on the properties described in Examples 4 to 6, the gel in Example 2 was preferred for the animal experiment of intra-articular injection.

44 New Zealand rabbits, each weighing no less than 1.8 kg, were acclimatized and then randomly divided into 5 groups, as shown in Table 7. Except for the sham-operated group, rabbits in other groups underwent anterior cruciate ligament transection surgery to induce mechanical instability, thereby establishing an OA model. Three days after the surgery, penicillin was injected to control infection. Six weeks after the surgery, rabbits were examined. When rabbits in groups A to D experienced knee joint stiffness and lameness and had positive anterior drawer test results, it was confirmed that the model was successfully established. After successful modeling was confirmed, according to Table 7, intra-articular injections were conducted. The group A was injected with 0.3 mL of the non-crosslinked carboxymethyl chitin (Libao Xi) once every two weeks with three injections in total. The group B received a single injection of 0.3 mL of the crosslinked carboxymethyl chitin in Example 2. The group C was injected with 0.3 mL of sodium hyaluronate (Synvisc) once every two weeks with three injections in total. The group D was injected with 0.3 mL of normal saline once every two weeks with three injections in total.

**Table 7 Animal grouping and treatment plan**

| Group | Sample name | Number of animals | Dose | Injection frequency |
|---|---|---|---|---|
| A | Non-crosslinked carboxymethyl chitin (Libao Xi) | 9 (n=18) | 0.3mL | Once every two weeks with three injections in total |
| B | Crosslinked carboxymethyl chitin (Example 2) | 9 (n=18) | 0.3mL | Single injection |
| C | Sodium hyaluronate (Synvisc) | 9 (n=18) | 0.3mL | Once every two weeks with three injections in total |
| D | Normal saline | 9 (n=18) | 0.3mL | Once every two weeks with three injections in total |
| E | Sham-operated group | 5 (n=10) | / | / |

At weeks 8, 12, and 18 after the modeling, animals in the groups A, B, C, and D were each sacrificed by injecting an overdose of an anesthetic through the marginal ear vein. Samples were collected through the original incision on the left hind limb for observation. Animals in the group E were sacrificed at week 18 after the modeling.

At each time point, the wear of the femoral condyle was observed in each group. Pathological changes on the articular surface of the femoral condyle were examined under a microscope, and gross specimen scoring was conducted. Scoring criteria: 0: smooth articular surface with normal color; 1: rough articular surface with small fissures and dull color; 2: eroded articular surface with cartilage defects reaching the surface and middle layers of cartilage; 3: ulceration of the articular surface with defects reaching the deep layer of cartilage; and 4: cartilage detachment with exposure of subchondral bone. Joints at each score in each group was counted and recorded in Table 8.

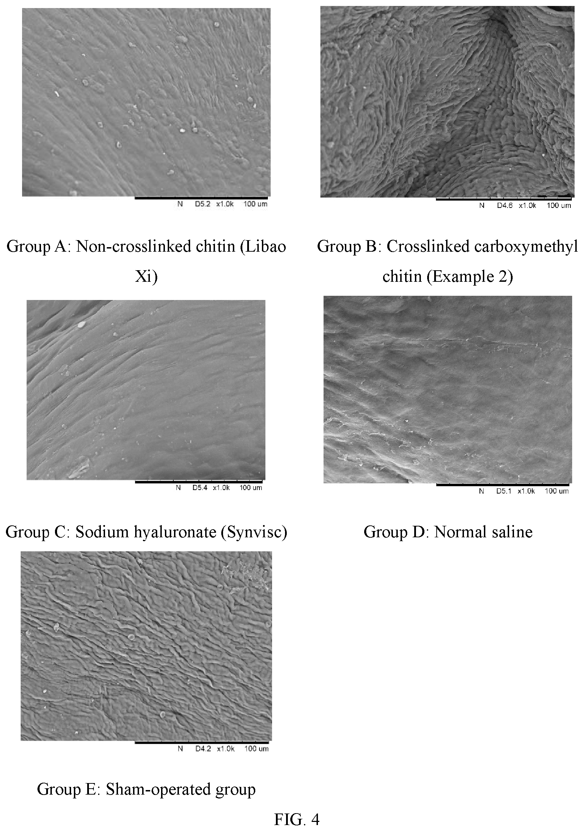

At week 18 after the surgery, a partial cartilage tissue was collected from the femoral condyle of the knee joint of each rabbit in each group and observed under a scanning electron microscope, as shown in FIG. 4.

At week 18 after the surgery, paraffin-embedded cartilage tissue sections with a thickness of 3 µm were prepared and subjected to routine hematoxylin and eosin (HE) staining and safranin O-fast green staining. Immunohistochemical staining was conducted for type II collagen. An expression level of type II collagen was calculated according to the method described by Pelletier (Pelletier JP, Lascau-Coman V, Jovanovic D, et al. Selective inhibition of inducible nitric oxide synthase in experimental osteoarthritis is associated with reduction in tissue levels of catabolic factors. J Rheumatol, 1999, 26 (9): 2002-2014) and expressed as a cell fraction (percentage of positive cells relative to total cells).

**Table 8 Pathological scores for femoral condyle articular surfaces of rabbits in each group at different time points**

| Group | At week 8 after the surgery | | | | | At week 12 after the surgery | | | | | At week 18 after the surgery | | | | |
|---|---|---|---|---|---|---|---|---|---|---|---|---|---|---|---|
| | **0** | **1** | **2** | **3** | **4** | **0** | **1** | **2** | **3** | **4** | **0** | **1** | **2** | **3** | **4** |
| **A** | 0 | 2 | 3 | 1 | 0 | 0 | 3 | 2 | 1 | 0 | 0 | 2 | 3 | 1 | 0 |
| **B** | 0 | 5 | 1 | 0 | 0 | 0 | 4 | 2 | 0 | 0 | 1 | 3 | 2 | 0 | 0 |
| **C** | 0 | 1 | 2 | 3 | 0 | 0 | 2 | 2 | 2 | 0 | 0 | 1 | 3 | 2 | 0 |
| **D** | 0 | 0 | 0 | 2 | 4 | 0 | 0 | 0 | 3 | 3 | 0 | 0 | 0 | 3 | 3 |
| **E** | - | - | - | - | - | - | - | - | - | - | 4 | 1 | 0 | 0 | 0 |

Scanning electron microscopy observation results of articular cartilage surfaces of rabbits in each group at week 19 after the surgery were shown in FIG. 4. According to macroscopic scanning electron microscopy observation results, the cartilage structure in the group E was nearly normal, cartilage surface structures underwent more severe wear in the groups A, B, and C than in the group E, and the group D underwent the most severe wear. Among the groups A, B, and C, the groups B and E had highly similar cartilage structures, which were better than the similar cartilage structures of the groups A and C.

HE staining: In the group A, the articular surface was rough, and there was a smaller number of chondrocytes than the group B. In the group B, the articular surface was intact, and the chondrocyte layers were visible. The groups C and A exhibited similar conditions, with a rough articular surface and a smaller number of chondrocytes than the group B. In the group D, the integrity of the articular surface was disrupted, there were obvious cracks and fibrosis, chondrocytes were atrophic and arranged in disarray, and the extracellular matrix was reduced compared to other groups. In the group E, the articular surface was intact and smooth, and chondrocytes had a normal morphology.

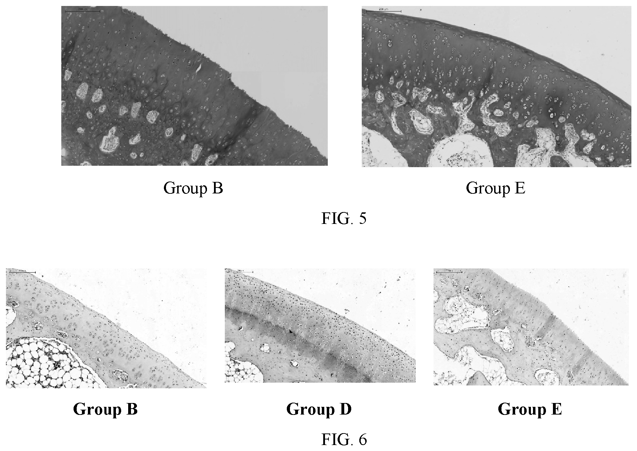

Safranin O-fast green staining: In the group A, the cartilage matrix was partially degraded, and safranin O staining was reduced compared to the group B. In the group B, the cartilage matrix was slightly degraded, and safranin O staining was slightly reduced compared to the group E (as shown in FIG. 5). The groups C and A exhibited similar conditions, where the cartilage matrix was partially degraded and safranin O staining was reduced compared with the group B. In the group D, the cartilage matrix was extensively degraded, and safranin O staining was significantly reduced compared to the group E. In the group E, the cartilage structure was regular, the cartilage matrix was abundant, and safranin O staining was the most pronounced.

Percentages of type II collagen-positive chondrocytes in the groups A, B, C, D, and E were 55.25%±6.94%, 75.20%±5.45%, 52.34%±6.79%, 41.25%±5.44%, and 81.33%±7.31%, respectively. Percentages of type II collagen-positive chondrocytes in the groups A, C, and D were significantly lower than a percentage of type II collagen-positive chondrocytes in the group E, and a percentage of type II collagen-positive chondrocytes in the group B was higher than a percentage of type II collagen-positive chondrocytes in the group D (as shown in FIG. 6), with statistically significant differences (P < 0.05). There was no statistically significant difference among the groups A, B, and C (P > 0.05).

The crosslinked carboxymethyl chitin can significantly ameliorate knee OA in rabbits, delay the pathological progression of OA, and effectively protect damaged cartilage. Moreover, the crosslinked carboxymethyl chitin requires fewer injections than the non-crosslinked carboxymethyl chitin (Libao Xi) and sodium hyaluronate (Synvisc), which is beneficial for improving patient compliance.

## Claims

1. A method for preparing a viscosupplement, wherein the viscosupplement comprises 0.5 wt% to 5 wt% of crosslinked carboxymethyl chitin and 0.5 wt% to 1.8 wt% of a buffer; the buffer is selected from the group consisting of a phosphate, a borate, a citrate, or any combination thereof; the viscosupplement has a pH of 7.0±0.5 and an osmotic pressure of 260 mOsm/kg to 400 mOsm/kg; the crosslinked carboxymethyl chitin comprises carboxymethyl chitin and a crosslinking agent; the crosslinking agent comprises two or more amino groups and is selected from the group consisting of chitosan oligosaccharide, lysine, polylysine, arginine, polyarginine, cystamine, spermidine, spermine, putrescine and a salt thereof, polyethylene glycol diamine, carnosine, an antimicrobial peptide, a collagen derived peptide, a cell binding peptide, an Arg-Gly-Asp (RGD) peptide, an RGD derived peptide, or any combination thereof; a degree of modification (MoD) of the crosslinked carboxymethyl chitin is 10% to 18%; and the method comprises:
a) dissolving the crosslinking agent to produce a crosslinking agent reaction solution, wherein the crosslinking agent reaction solution comprises a buffer system with a pH of 5 to 9;
b) adding the carboxymethyl chitin to the crosslinking agent reaction solution, and mixing, wherein a molar ratio of carboxyl groups in the carboxymethyl chitin to amino groups in the crosslinking agent is 3:2 to 1:2;
c) adding a condensing agent to initiate an amidation reaction between the carboxymethyl chitin and the crosslinking agent, wherein a molar ratio of the carboxyl groups in the carboxymethyl chitin to the condensing agent is 4:1 to 1:2, and the condensing agent is selected from the group consisting of 1-ethyl-3-(3-dimethylaminopropyl)carbodiimide (EDC), 4-(4,6-dimethoxy-1,3,5-triazine-2-yl)-4-methyl-morpholinium chloride (DMTMM), 1-ethyl-3-(3-dimethylaminopropyl)carbodiimide hydrochloride (EDCI), 2-chloro-4,6-dimethoxy-1,3,5-triazine (CDMT), or a combination thereof;
d) diluting a resulting gel with the buffer to 0.5% to 5% (w/w); and
e) conducting dialysis with the buffer to produce the viscosupplement,
wherein the method does not comprise use of ethanol, acetone, or methanol.

2. The method according to claim 1, wherein in the step a), the buffer system comprises one or a combination of salts of a chloride ion, a borate ion, an acetate ion, a sulfate ion, a phosphate ion, a citrate ion, and a hydrogen phosphate ion; and/or
a total molar concentration of anions in the buffer system is 0.02 M to 2.0 M.

3. The method according to claim 2, wherein a pH of the buffer system is 6 to 8.

4. The method according to claim 1, wherein in the step b),
the carboxymethyl chitin has a degree of deacetylation of no more than 20%, a degree of carboxymethyl substitution of 1.0 to 2.0, a molecular weight of no less than 300 kDa, and a dynamic viscosity of no less than 4,000 mPa·s (at 25 mg/mL);
a mass concentration of the carboxymethyl chitin is 1% to 10% (w/w); and/or
a temperature is 10±5°C.

5. The method according to claim 4, wherein the mass concentration of the carboxymethyl chitin is 2.5% to 6% (w/w).

6. The method according to claim 1, wherein in the step c),
the condensing agent is an aqueous solution, and/or a concentration of the condensing agent is 2% to 20% (w/w);
a reaction time is 24 h to 96 h; and/or
a temperature is 10±5°C.

7. The method according to claim 6, wherein the reaction time is 48 h to 72 h.

8. The method according to claim 1, wherein in the step e),
a dialysis concentration is 1.0% to 5.4% (w/w); and/or
a dialysis time is 72 h to 180 h.

9. The method according to claim 1, further comprising: f) pre-filling the viscosupplement obtained in the step e) into a glass syringe, and conducting high-temperature sterilization.

10. The method according to claim 9, wherein
the pre-filling comprises pre-filled sealing, and/or a filling volume is 1 mL to 10 mL; and/or
the high-temperature sterilization comprises moist-heat sterilization; the high-temperature sterilization is conducted at 110°C to 130°C; and/or a dynamic viscosity of the viscosupplement after the high-temperature sterilization is 60% to 118% of a dynamic viscosity of the viscosupplement before the high-temperature sterilization.
